# EUROPEAN PATENT APPLICATION

(11) **EP 2 832 286 A1**
(43) Date of publication of application: **04.02.2015**
(21) Application number: 14178315.9
(22) Date of filing: 24.07.2014
(51) Int. Cl.: A61B 5/00, A61B 5/1455, G01N 29/24

(54) **Photoacoustic imaging apparatus comprising a plurality of LED elements**

(30) Priority: 31.07.2013 JP 2013159081
(71) Applicant: Funai Electric Co., Ltd., Daito-shi, Osaka 574-0013 (JP)
(72) Inventor: Agano, Toshitaka, Daito-shi Osaka 574-0013 (JP)
(74) Representative: Grünecker, Kinkeldey, Stockmair & Schwanhäusser

(57) **Abstract**

In a photoacoustic imaging apparatus (100), a detecting portion (20) includes a light source portion (21) having a plurality of LED elements (21a, 21b), an ultrasonic detector (24) arranged in the vicinity of the light source portion, configured to detect an acoustic wave as an ultrasonic wave generated by absorption of light emitted from the light source portion to a test object, and a light source drive circuit arranged in the vicinity of the light source portion, driving the light source portion.

## Description

### BACKGROUND OF THE INVENTION

### Field of the Invention

The present invention relates to a photoacoustic imaging apparatus, and more particularly, it relates to a photoacoustic imaging apparatus including a light source portion emitting light to a test object and an ultrasonic detector detecting an acoustic wave as an ultrasonic wave generated by a target in the test object.

### Description of the Background Art

A photoacoustic imaging apparatus or the like including a light source portion emitting light to a test object and an ultrasonic detector detecting an acoustic wave as an ultrasonic wave generated by a target in the test object is known in general, as disclosed in Japanese Patent Laying-Open No. 6-296612 (1994).

Japanese Patent Laying-Open No. 6-296612 discloses a photoacoustic computed tomography apparatus (photoacoustic imaging apparatus) including a laser beam source generating probe light, an optical cable guiding the probe light to an object to be measured (test object), acoustic signal detectors (ultrasonic detector) arranged on a surface of the object to be measured, detecting the intensity of only an acoustic signal (acoustic wave) of the probe light, and a computer system computing a material distribution in the object to be measured by processing detected acoustic signal information, indicating the timing of generating the probe light to the laser beam source. In this photoacoustic computed tomography apparatus, an acoustic signal is generated from a target absorbing the energy of light in the object to be measured, and the acoustic signal propagates inside the object to be measured when the light is emitted from the laser beam source to the object to be measured. Furthermore, this acoustic signal is detected by the acoustic signal detectors, and the computer system performs signal processing on the basis of the detected acoustic signal, whereby the target generating the acoustic signal is visualized (imaged).

This photoacoustic imaging apparatus is subjected to active research in a medical field as an imaging apparatus for obtaining information in a living body.

In the photoacoustic computed tomography apparatus described in Japanese Patent Laying-Open No. 6-296612, however, a high-power solid-state laser is conceivably employed as a light source in order to obtain a high signal-to-noise ratio. Highly accurate alignment (arrangement) of members is required to employ this solid-state laser as a light source, and an optical surface plate for suppressing property fluctuation resulting from vibration of an optical system and a strong housing are necessary. Consequently, an apparatus for generating a laser beam is increased in size and complicated in structure, and hence it is difficult to downsize and simplify the photoacoustic computed tomography apparatus.

### SUMMARY OF THE INVENTION

The present invention has been proposed in order to solve the aforementioned problem, and an object of the present invention is to provide a photoacoustic imaging apparatus capable of being downsized and simplified.

A photoacoustic imaging apparatus according to an aspect of the present invention includes a detecting portion configured to be capable of emitting light to a test object, detecting an acoustic wave generated by absorption of the light that is emitted by a target in the test object, and a signal processing portion processing and imaging a signal detected by the detecting portion, and the detecting portion is arranged close to the test object and includes a light source portion having a plurality of LED elements emitting the light to the test object, an ultrasonic detector arranged in the vicinity of the light source portion, configured to detect the acoustic wave as an ultrasonic wave generated by the absorption of the light emitted from the light source portion to the test object, and a light source drive circuit arranged in the vicinity of the light source portion, driving the light source portion.

As hereinabove described, the photoacoustic imaging apparatus according to the aspect of the present invention is provided with the light source portion having the plurality of LED elements emitting the light to the test object, whereby no optical surface plate for suppressing property fluctuation resulting from vibration of an optical system (device peripheral to a solid-state laser beam source) or strong housing is necessary unlike the case where a solid-state laser is employed as a light source, and hence the light source portion can be downsized. Furthermore, the light source portion is provided in the detecting portion arranged close to the test object, whereby the light source portion and the detecting portion are integrated, so that the simple structure can be achieved. Thus, the photoacoustic imaging apparatus can be downsized and simplified (compactified). In addition, the light source drive circuit is arranged in the vicinity of the light source portion in the detecting portion, whereby the light source drive circuit is close to the light source portion, so that generation of noise resulting from a drive signal of the light source drive circuit can be suppressed. Thus, a bad influence (disturbance of the signal or the like) of the noise generated by the drive signal on a device such as the ultrasonic detector can be suppressed.

In the aforementioned photoacoustic imaging apparatus according to the aspect, the ultrasonic detector preferably has an ultrasonic vibrating element detecting the acoustic wave as the ultrasonic wave, in a plan view, the ultrasonic vibrating element is preferably arranged in a central portion of the detecting portion, and the light source portion is preferably arranged adjacent to the ultrasonic vibrating element. According to this structure, the light source portion can be easily arranged at a position in the vicinity of the ultrasonic detector having the ultrasonic vibrating element.

In the aforementioned photoacoustic imaging apparatus according to the aspect, the ultrasonic vibrating element of the ultrasonic detector is preferably arranged to extend along a first direction, and the light source portion having the LED elements is preferably arranged in the vicinity of the ultrasonic detector to extend along the first direction. According to this structure, both the ultrasonic vibrating element detecting the acoustic wave as the ultrasonic wave and the light source portion having the LED elements are arranged along the first direction, and hence the light can be emitted from the light source portion to the test object along the arrangement of the ultrasonic vibrating element. Consequently, the acoustic wave along the first direction can be generated, and hence the ultrasonic vibrating element along the first direction can efficiently detect the generated acoustic wave.

In this case, the light source portion is preferably arranged in the vicinity of the ultrasonic detector to sandwich the ultrasonic vibrating element from a first side of a second direction orthogonal to the first direction and a second side thereof. According to this structure, the light along the arrangement of the ultrasonic vibrating element can be emitted to the test object from both sides of the ultrasonic vibrating element detecting the acoustic wave, and hence the amount of light emitted to the test object can be easily increased. Consequently, the larger acoustic wave along the first direction can be generated, and hence the acoustic wave generated by the ultrasonic vibrating element along the first direction can be more efficiently detected.

In the aforementioned structure in which the ultrasonic vibrating element and the light source portion having the LED elements are arranged to extend along the first direction, the light source portion is preferably arranged such that a mounting surface mounted with the LED elements is inclined toward the ultrasonic vibrating element. According to this structure, the light from the light source portion can be emitted in a direction in which the ultrasonic vibrating element is arranged, and hence the light can be reliably emitted to the test object in the vicinity of the ultrasonic vibrating element. Consequently, the detectable acoustic wave can be more efficiently generated.

In the aforementioned photoacoustic imaging apparatus according to the aspect, the detecting portion preferably further includes a light guide portion guiding the light from the light source portion included in the detecting portion to the test object. According to this structure, a loss of the light between the light source portion and the test object can be reduced, and hence the loss of the light is reduced, so that the use efficiency of the light emitted from the light source portion can be improved.

In this case, the light source portion is preferably arranged to extend along a first direction, and the light guide portion is preferably formed to extend along the first direction in which the light source portion extends. According to this structure, both the light source portion emitting the light and the light guide portion guiding the light from the light source portion to the test object are arranged along the first direction, and hence the light can be reliably emitted from the light source portion to the light guide portion. Consequently, the loss of the light between the light source portion and the test object can be reduced, and hence the loss of the light is reduced, so that the use efficiency of the light emitted from the light source portion can be improved.

In the aforementioned photoacoustic imaging apparatus according to the aspect, the detecting portion preferably further includes a drive power supply portion arranged in the vicinity of the light source portion of the detecting portion, supplying power to the light source portion. According to this structure, the drive power supply portion is arranged in the vicinity of the light source portion, and hence the drive power supply portion is close to the light source portion, so that generation of noise can be suppressed when the drive power supply portion supplies power to the light source portion. Consequently, a bad influence (disturbance of the signal or the like) of the noise generated when the power is supplied on a device such as the ultrasonic detector can be further suppressed.

In the aforementioned structure in which the detecting portion includes the light guide portion guiding the light from the light source portion to the test object, the detecting portion preferably further includes a condensing portion arranged between the light source portion and the light guide portion, condensing the light from the light source portion onto the light guide portion. According to this structure, the light from the light source portion can be reliably condensed onto the light guide portion, and hence the use efficiency of the light is increased, and the amount of light emitted to the test object can be easily ensured.

In the aforementioned photoacoustic imaging apparatus according to the aspect, the plurality of LED elements of the light source portion preferably include a first LED element arranged in the vicinity of the ultrasonic detector, emitting the light having a first wavelength and a second LED element arranged in the vicinity of the ultrasonic detector, emitting the light having a second wavelength. According to this structure, two types of wavelengths are employed, whereby the amount of detected information can be increased (two types of acoustic waves can be obtained), and hence more various image information (detection signals) can be obtained, as compared with the case where one wavelength is employed.

In this case, the light source drive circuit is preferably configured to drive the first LED element and the second LED element such that an output of the light having the first wavelength and an output of the light having the second wavelength are equal to each other. According to this structure, even when the conversion efficiency of the light output with respect to power of the first LED element and the conversion efficiency of the light output with respect to power of the second LED element are different from each other, the outputs of the light having two types of wavelengths can be equal to each other, and hence the intensity of the light having two types of wavelengths emitted to the test object can be matched.

In the aforementioned structure in which the light source portion includes the first LED element and the second LED element, the light source portion preferably has a plurality of first LED elements and a plurality of second LED elements, the numbers of which are adjusted according to the ratio of a maximum output of the light emitted from the first LED elements and a maximum output of the light emitted from the second LED elements such that a total output of the light from the plurality of first LED elements and a total output of the light from the plurality of second LED elements are equal to each other. According to this structure, even when the conversion efficiency of the light output with respect to the power of the first LED elements and the conversion efficiency of the light output with respect to the power of the second LED elements are different from each other, the number of the plurality of first LED elements and the number of the plurality of second LED elements are adjusted according to the ratio of the maximum outputs, whereby the outputs of the light having two types of wavelengths can be equal to each other, and hence the intensity of the light having two types of wavelengths emitted to the test object can be matched.

In the aforementioned structure in which the light source portion includes the first LED element and the second LED element, the first LED element and the second LED element are preferably arranged in the light source portion such that the distribution shapes of the light emitted from a plurality of first LED elements and a plurality of second LED elements are identical to each other. According to this structure, the distribution shapes of the light emitted from the plurality of first LED elements and the plurality of second LED elements can be identical to each other, and hence variations in emission of the light having the first and second wavelengths to the target of the test object resulting from the distribution shapes of the light from the first LED elements and the second LED elements can be suppressed.

In this case, the light source portion is preferably configured such that the distribution shapes of the light having the first wavelength and the second wavelength emitted from the plurality of LED elements are identical to each other by alternately arranging the plurality of first LED elements formed of a single group or a plurality of groups and the plurality of second LED elements formed of a single group or a plurality of groups. According to this structure, the first LED elements and the second LED elements are alternately arranged, whereby not only the distribution shapes of the light from the first LED elements and the second LED elements but also the distribution positions of the light having the first wavelength and the second wavelength with respect to the light source portion can be identical to each other, and hence the variations in the emission of the light having the first and second wavelengths to the target of the test object resulting from the distribution shapes and the distribution positions of the light from the first LED elements and the second LED elements can be reliably suppressed.

In the aforementioned photoacoustic imaging apparatus according to the aspect, the ultrasonic detector is preferably configured to generate the ultrasonic wave to the test object and detect the ultrasonic wave reflected by the test object, and the signal processing portion is preferably configured to process and image the signal on the basis of detection results of both the acoustic wave generated by the absorption of the light by the target in the test object and the ultrasonic wave reflected by the test object. According to this structure, the signal processing portion can image the detection signals of not only the acoustic wave generated by the absorption of the light by the target in the test object but also the ultrasonic wave reflected by the test object, and hence an image in the test object can be more accurately obtained.

According to the present invention, as hereinabove described, the photoacoustic imaging apparatus capable of being downsized and simplified can be provided.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a diagram showing the overall structure of a photoacoustic imaging apparatus according to a first embodiment of the present invention;
Fig. 2 is a block diagram showing the overall structure of the photoacoustic imaging apparatus according to the first embodiment of the present invention;
Fig. 3 is a side elevational view of a detecting portion of the photoacoustic imaging apparatus according to the first embodiment of the present invention;
Fig. 4 is a plan view of the detecting portion of the photoacoustic imaging apparatus according to the first embodiment of the present invention;
Fig. 5 is a diagram showing the arrangement of LED elements on a light source portion of the photoacoustic imaging apparatus according to the first embodiment of the present invention;
Fig. 6 is a diagram showing a modification of the arrangement of the LED elements on the light source portion of the photoacoustic imaging apparatus according to the first embodiment of the present invention;
Fig. 7 is a block diagram showing the overall structure of a photoacoustic imaging apparatus according to a second embodiment of the present invention;
Fig. 8 illustrates a state before an ultrasonic probe is mounted on a light source unit of the photoacoustic imaging apparatus according to the second embodiment of the present invention;
Fig. 9 illustrates a state where the ultrasonic probe is mounted on the light source unit of the photoacoustic imaging apparatus according to the second embodiment of the present invention;
Fig. 10 is a diagram showing the arrangement of LED elements on a light source portion of the photoacoustic imaging apparatus according to the second embodiment of the present invention;
Fig. 11 is a diagram for illustrating the structure of a photoacoustic imaging apparatus according to a third embodiment of the present invention; and
Fig. 12 is a diagram for illustrating a modification of the first embodiment of the present invention.

### DESCRIPTION OF THE PREFERRED EMBODIMENTS

Embodiments of the present invention are hereinafter described with reference to the drawings.

### (First Embodiment)

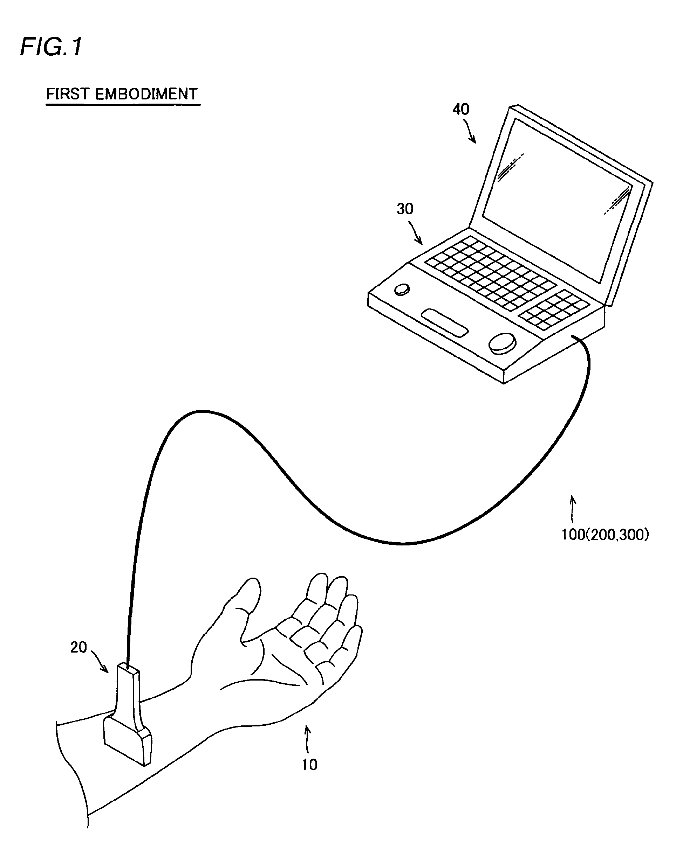

The structure of a photoacoustic imaging apparatus 100 according to a first embodiment of the present invention is now described with reference to Figs. 1 to 6.

The photoacoustic imaging apparatus 100 according to the first embodiment of the present invention includes a detecting portion 20 configured to acquire image information in a test object 10, a signal processing portion 30 processing and imaging a signal detected by the detecting portion 20, and an image display portion 40 configured to display an image processed by the signal processing portion 30, as shown in Fig. 1.

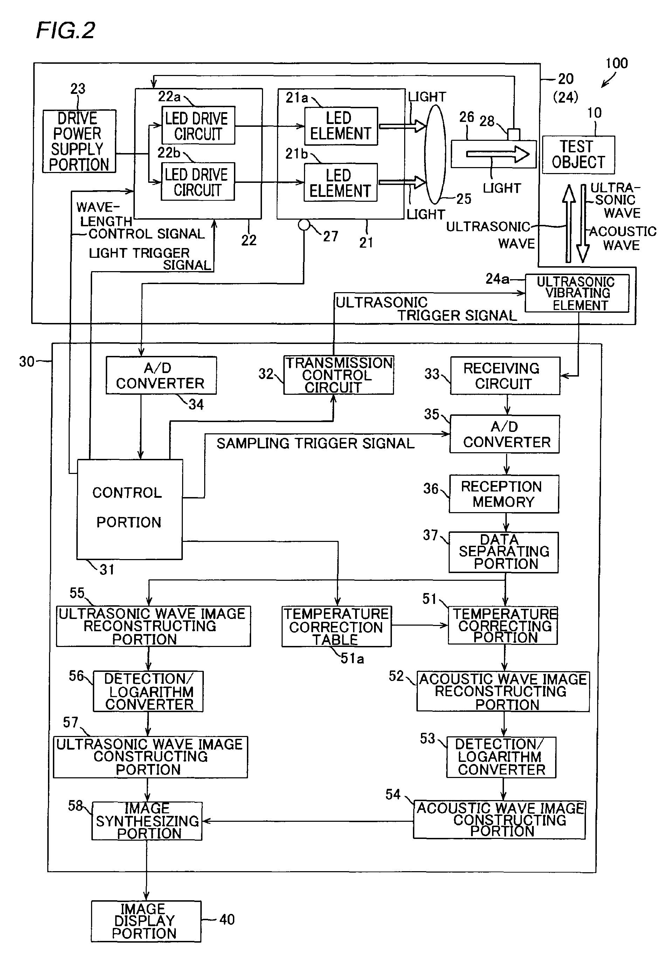

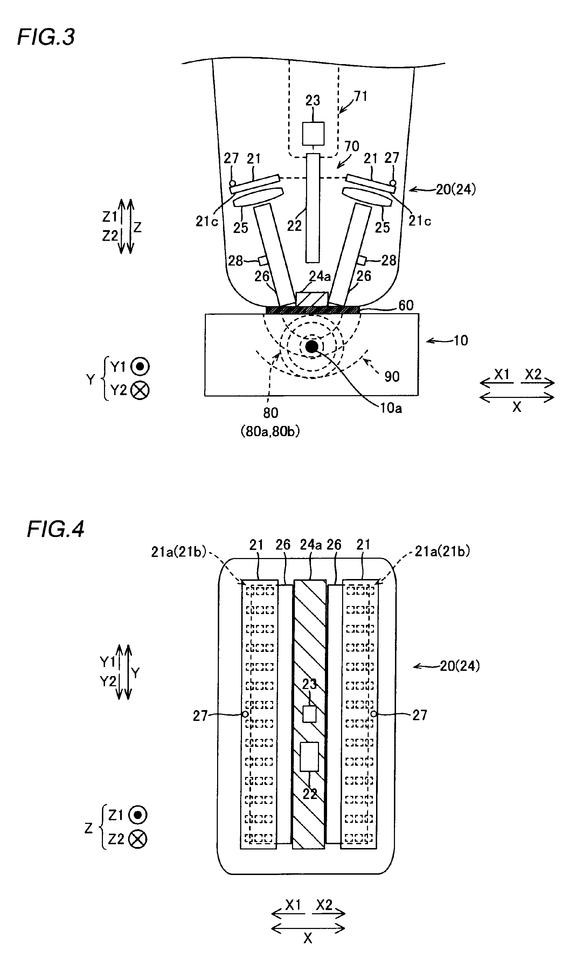

According to the first embodiment, the detecting portion 20 includes a light source portion 21 emitting light to the test object 10, a light source drive circuit 22 transmitting a drive signal to the light source portion 21, and a drive power supply portion 23 supplying power to the light source portion 21 through the light source drive circuit 22, as shown in Fig. 2. The detecting portion 20 includes an ultrasonic probe 24 having a function of detecting an acoustic wave 80 (see Fig. 3) as an ultrasonic wave generated by absorption of the light by a light absorber 10a (see Fig. 3) in the test object 10 and a function of generating an ultrasonic wave 90 (see Fig. 3) to the test object 10 and detecting the ultrasonic wave 90 reflected by the test object 10. The detecting portion 20 further includes a condensing lens 25 condensing the light from the light source portion 21 and a light guide portion 26 guiding the light condensed by the condensing lens 25 to the vicinity of the test object 10. The detecting portion 20 further includes a temperature sensor 27 arranged on the light source portion 21, detecting the temperature of the light source portion 21 and a light sensor 28 arranged on the light guide portion 26, detecting the light reflected by the test object 10. In Fig. 2, only a set of the light source portion 21, the condensing lens 25, the light guide portion 26, the temperature sensor 27, and the light sensor 28 is shown for the convenience of illustration, but two sets of the aforementioned components are actually arranged in the detecting portion 20, as shown in Fig. 3. The ultrasonic probe 24 is an example of the "ultrasonic detector" in the present invention. The condensing lenses 25 are examples of the "condensing portion" in the present invention. The light absorber 10a is an example of the "target" in the present invention.

In this description, the ultrasonic wave denotes an acoustic wave (elastic wave) having such a high frequency that a person with normal hearing does not have a sensation of hearing and an acoustic wave (elastic wave) of at least about 16000 Hz. In this description, an ultrasonic wave generated by absorption of light by the light absorber 10a in the test object 10 is distinguished as the "acoustic wave" from an ultrasonic wave generated by the ultrasonic probe 24, reflected by the test object 10 as the "ultrasonic wave" for the convenience of description.

The light source portions 21 each have a plurality of LED elements 21a emitting light having a first wavelength and a plurality of LED elements 21b emitting light having a second wavelength. These LED elements 21a and LED elements 21b have maximum outputs different from each other. The first wavelength and the second wavelength are arbitrarily selected according to an object to be measured. In the case where oxygenated hemoglobin and reduced hemoglobin in the blood are detected, for example, the first wavelength is set to about 760 nm, and the second wavelength is set to about 850 nm. In this case, utilizing the inversion near a wavelength of about 800 nm of the magnitude relationship between the absorption spectrum of the oxygenated hemoglobin and the absorption spectrum of the reduced hemoglobin, a difference between the intensity of an acoustic wave signal detected with the light having the first wavelength and the intensity of an acoustic wave signal detected with the light having the second wavelength is calculated, whereby which hemoglobin is more contained in the blood can be detected. Thus, an artery or a vein in the test object 10 can be determined and imaged. The LED elements 21a are examples of the "first LED element" in the present invention. The LED elements 21b are examples of the "second LED element" in the present invention.

The light source drive circuit 22 has an LED drive circuit 22a driving the LED elements 21a and an LED drive circuit 22b driving the LED elements 21b. The light source drive circuit 22 is configured to supply power for light emission to the LED elements 21a and the LED elements 21b through the LED drive circuit 22a and the LED drive circuit 22b, respectively. The LED drive circuit 22a and the LED drive circuit 22b are configured to supply different drive power to the LED elements 21a and the LED elements 21b. The light source drive circuit 22 is configured to drive the LED elements 21a and the LED elements 22b such that an output of the light having the first wavelength and an output of the light having the second wavelength are substantially equal to each other.

The signal processing portion 30 includes a control portion 31 configured to control components of the signal processing portion 30 and the light source drive circuit 22, a transmission control circuit 32 transmitting an ultrasonic trigger signal to the ultrasonic probe 24 on the basis of the control performed by the control portion 31, a receiving circuit 33 configured to receive signals of the acoustic wave 80 and the ultrasonic wave 90 detected by the ultrasonic probe 24, and an A/D converter 34 converting temperature signals acquired by the temperature sensors 27 into a digital signal. The signal processing portion 30 further includes an A/D converter 35 converting the detected signals of the acoustic wave 80 and the ultrasonic wave 90 into digital signals, a reception memory 36 storing the detected signals of the acoustic wave 80 and the ultrasonic wave 90, and a data separating portion 37 separating the detected signals of the acoustic wave 80 and the ultrasonic wave 90 from each other. The signal processing portion 30 further includes a temperature correcting portion 51 correcting the separated signal of the acoustic wave 80 employing a temperature correction table 51a, an acoustic wave image reconstructing portion 52 reconstructing the temperature-corrected signal of the acoustic wave 80 as an image, a detection/logarithm converter 53 performing waveform processing upon output of the acoustic wave image reconstructing portion 52, and an acoustic wave image constructing portion 54 constructing a tomographic image in the test object 10 based on the detected signal of the acoustic wave 80. The signal processing portion 30 further includes an ultrasonic wave image reconstructing portion 55 reconstructing the signal of the ultrasonic wave 90 reflected from the test object 10 as an image, a detection/logarithm converter 56 performing waveform processing upon output of the ultrasonic wave image reconstructing portion 55, and an ultrasonic wave image constructing portion 57 constructing a tomographic image in the test object 10 based on the detected signal of the ultrasonic wave 90. The signal processing portion 30 further includes an image synthesizing portion 58 synthesizing the image constructed by the acoustic wave image constructing portion 54 and the image constructed by the ultrasonic wave image constructing portion 57 and is configured to output an image synthesized by the image synthesizing portion 58 to the image display portion 40.

The temperature correction table 51a is a table for temperature correction prepared on the basis of a change in a wavelength with respect to a change in the temperature of the LED elements 21a and the LED elements 21b and a change in an acoustic wave signal of the light absorber 10a (see Fig. 3) with respect to the change in the wavelength, with which the change in the acoustic wave signal of the light absorber 10a with respect to the change in the temperature can be calculated.

According to the first embodiment, the ultrasonic probe 24 has an ultrasonic vibrating element 24a (shown by hatching) capable of emitting the ultrasonic wave 90 to the test object 10, detecting the acoustic wave 80 and the ultrasonic wave 90 reflected by the test object 10, as shown in Fig. 3. In the detecting portion 20, the ultrasonic vibrating element 24a is arranged in the vicinity of the test object 10. In other words, the ultrasonic vibrating element 24a is arranged in the vicinity of an end face closer to the test object 10 (on a Z2 side) in the detecting portion 20. According to this first embodiment, each component such as the light source portions 21 is arranged in the ultrasonic probe 24, whereby the detecting portion 20 is formed.

The detecting portion 20 includes the two light source portions 21, the two condensing lenses 25, the two light guide portions 26, the two temperature sensors 27, and the two light sensors 28 (in pairs), and the aforementioned components in pairs are arranged to sandwich the ultrasonic vibrating element 24a therebetween. In the detecting portion 20, the light source drive circuit 22 and the drive power supply portion 23 are arranged between the two light source portions 21. In the detecting portion 20, the light source drive circuit 22 is arranged in the vicinity of the light source portions 21. The detecting portion 20 is configured such that the two light source portions 21, the light source drive circuit 22, and the drive power supply portion 23 are electrically connected to each other by electrical wires 70 (shown by broken lines) therein. Furthermore, the detecting portion 20 is configured such that the light source drive circuit 22 receives a signal from the signal processing portion 30 through a wire 71 (shown by a broken line).

The detecting portion 20 is configured to perform detection in a state where a transparent gel layer 60 (shown by hatching) transmitting light intervenes between a surface of the detecting portion 20 closer to the test object 10 (on the Z2 side), on which the ultrasonic vibrating element 24a is arranged and the test object 10. This gel layer 60 has a refractive index substantially equal to that of a surface of the test object 10 and is configured to suppress reflection of the light emitted from the light source portions 21 to the test object 10 by the surface of the test object 10. The gel layer 60 also serves as a propagating substance for efficiently propagating the acoustic wave 80 generated by the light absorber 10a of the test object 10 and the ultrasonic wave 90 reflected by the test object 10 to the ultrasonic vibrating element 24a.

According to the first embodiment, the ultrasonic vibrating element 24a as a whole is arranged to extend along a direction Y, as shown in Fig. 4. The ultrasonic vibrating element 24a is configured such that a plurality of ultrasonic vibrating elements that are unshown are arranged along the direction Y. Furthermore, the ultrasonic vibrating element 24a is arranged in a substantially central portion of the detecting portion 20. The direction Y is an example of the "first direction" in the present invention.

The two light source portions 21 are arranged to sandwich the ultrasonic vibrating element 24a from a first side (X1 side) of a direction X orthogonal to the direction Y and a second side (X2 side) thereof, as shown in Fig. 3. The two light source portions 21 are arranged such that mounting surfaces 21c mounted with the LED elements 21a and the LED elements 21b (see Fig. 2) are inclined toward the ultrasonic vibrating element 24a. The light source portions 21 are configured to emit the light from the LED elements 21a and the LED elements 21b to the test object 10 in the vicinity of the ultrasonic vibrating element 24a. The light source portions 21 are arranged in the vicinity of the ultrasonic vibrating element 24a to extend along the direction Y, as shown in Fig. 4. The direction X is an example of the "second direction" in the present invention.

The condensing lenses 25 are arranged between the light source portions 21 and the light guide portions 26 to cover the mounting surfaces 21c of the light source portions 21, as shown in Fig. 3. The condensing lenses 25 are configured to condense light onto the central sides of the condensing lenses 25 by refracting light incident from the light source portions 21 and emit the condensed light to the light guide portions 26.

The light guide portions 26 each are in the form of a substantially flat plate extending along the direction Y, as shown in Figs. 3 and 4. The light guide portions 26 are made of transparent resin such as acrylic resin or polycarbonate resin. Incidence ends of the light guide portions 26 on a Z1 side on which light is incident are arranged in the vicinity of the condensing lenses 25, and emission ends of the light guide portions 26 on the Z2 side from which light is emitted are arranged in the vicinity of the end face of the detecting portion 20 closer to the test object 10 (on the Z2 side).

The light sensors 28 are configured to detect the light emitted from the light source portions 21 and reflected by the test object 10. The light sensors 28 are configured to be directly connected to the light source drive circuit 22. The light source drive circuit 22 is configured to stop emission of light from the light source portions 21 when the amount of reflection of light detected by the light sensors 28 is smaller than a prescribed amount. In other words, the light source drive circuit 22 is configured to stop the mission of light from the light source portions 21 when the detecting portion 20 is separated from the test object 10 and a detectable amount of reflection of light is reduced.

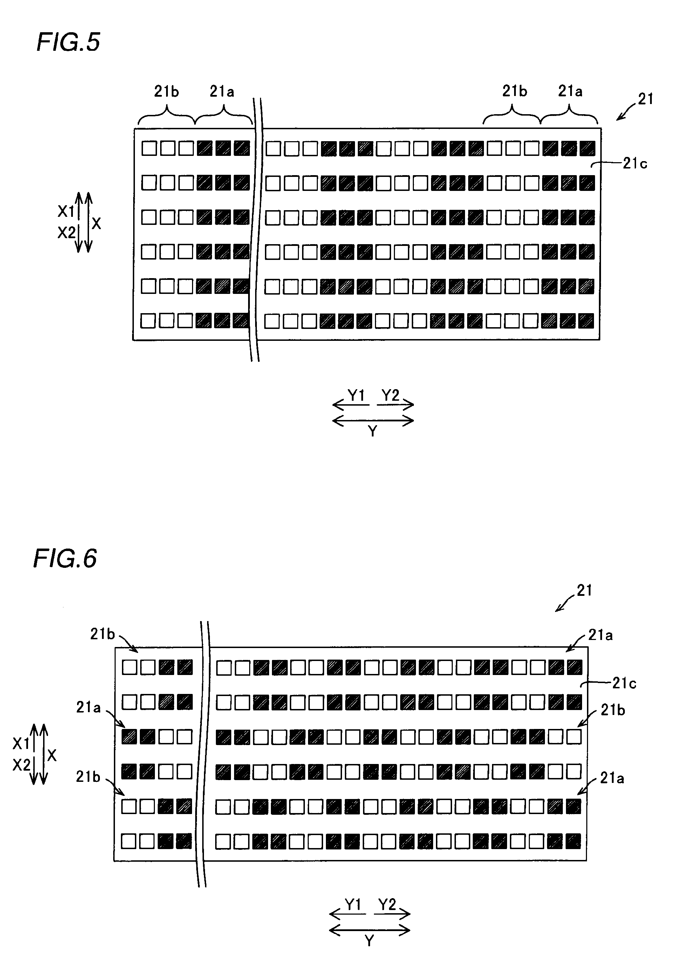

According to the first embodiment, the light source portions 21 are configured such that the plurality of LED elements 21a (shown by hatching) and the plurality of LED elements 21b are alternately lined, as shown in Fig. 5. Specifically, a line of the LED elements 21a formed of 3 x 6 LED elements 21a and a line of the LED elements 21b formed of 3 x 6 LED elements 21b similarly are alternately arranged on the mounting surfaces 21c of the light source portions 21.

The arrangement of the LED elements 21a and the LED elements 21b according to the first embodiment is not restricted to this, so far as the plurality of LED elements 21a and the plurality of LED elements 21b are alternately arranged on the mounting surfaces 21c. Arrangement shown in Fig. 6 may alternatively be employed, for example. Specifically, a group formed of 2 x 2 LED elements 21a and a group formed of 2 x 2 LED elements 21b may be alternately arranged in a lattice pattern. Furthermore, in the aforementioned arrangement of a group of N x N LED elements 21a and a group of N x N LED elements 21b, N may be a number other than 2. For the convenience of illustration, the middle of the light source portions 21 is omitted in Figs. 5 and 6. The arrangement of the LED elements 21a and the LED elements 21b of the two light source portions 21 is preferably substantially the same.

The detection operation of the photoacoustic imaging apparatus 100 according to the first embodiment of the present invention is now described with reference to Figs. 2 and 3.

First, a wavelength control signal is transmitted from the control portion 31 of the signal processing portion 30 to the light source drive circuit 22. The light source drive circuit 22 receiving the wavelength control signal selects either the LED elements 21a or the LED elements 21b on the basis of the received wavelength control signal. Then, the control portion 31 transmits a light trigger signal, and the light source drive circuit 22 receiving the light trigger signal transmits the drive signal to the selected LED elements 21a or LED elements 21b. The LED elements 21a or LED elements 21b receiving the drive signal emit the light to the test object 10 through the condensing lenses 25 and the light guide portions 26. The light emitted to the test object 10 propagates inside the test object 10 and is absorbed by the light absorber 10a. The light absorber 10a absorbs the light thereby generating the acoustic wave 80 (see Fig. 3) as the ultrasonic wave. Thereafter, the acoustic wave 80 (80a) propagating inside the test object 10 reaches the ultrasonic vibrating element 24a and is detected as the acoustic wave signal. Following the aforementioned operations, the non-selected LED elements 21a or LED elements 21b emit the light in the same order, and the acoustic wave 80 (80b) is detected.

After the acoustic waves 80a and 80b are detected, the control portion 31 transmits the ultrasonic trigger signal to the ultrasonic probe 24 through the transmission control circuit 32, as shown in Fig. 2. The ultrasonic probe 24 receiving the ultrasonic trigger signal generates the ultrasonic wave 90 from the ultrasonic vibrating element 24a to the test object 10, as shown in Fig. 3. Thereafter, the ultrasonic wave 90 reflected inside the test object 10 reaches the ultrasonic vibrating element 24a and is detected as an ultrasonic signal.

When the first light rigger signal is transmitted and the light is emitted from the light source portions 21, the control portion 31 starts to transmit a sampling trigger signal to the A/D converter 35, as shown in Fig. 2. The A/D converter 35 receiving the sampling trigger signal samples a detection signal of the ultrasonic probe 24 received by the receiving circuit 33. This sampling operation is continuously performed also on the signal of the ultrasonic wave 90 reflected inside the test object 10 after the signals of the acoustic waves 80a and 80b are sampled. Furthermore, this sampling operation may be performed with a prescribed sampling period in synchronization with an A/D clock signal externally input, for example.

The aforementioned detection operation is sequentially performed while the detecting portion 20 scans the surface of the test object 10. Consequently, the signal processing portion 30 synthesizes an image in the test object 10 on the basis of the sampled signals of the acoustic waves 80a and 80b and the signal of the ultrasonic wave 90, and the image in the test object 10 synthesized by the image display portion 40 is displayed.

According to the first embodiment, the following effects can be obtained.

According to the first embodiment, as hereinabove described, the photoacoustic imaging apparatus 100 is provided with the light source portions 21 having the plurality of LED elements 21a and the plurality of LED elements 21b emitting the light to the test object 10, whereby no optical surface plate for suppressing property fluctuation resulting from vibration of an optical system (device peripheral to a solid-state laser beam source) or strong housing is necessary unlike the case where a solid-state laser is employed as a light source, and hence the light source portions 21 can be downsized. Furthermore, the light source portions 21 are provided in the detecting portion 20 arranged close to the test object 10, whereby the light source portions 21 and the detecting portion 20 are integrated, so that the simple structure can be achieved. Thus, the photoacoustic imaging apparatus 100 can be downsized and simplified (compactified). In addition, the light source drive circuit 22 is arranged in the vicinity of the light source portions 21 in the detecting portion 20, whereby the light source drive circuit 22 is close to the light source portions, so that generation of noise resulting from the drive signal of the light source drive circuit 22 can be suppressed. Thus, a bad influence (disturbance of the signal or the like) of the noise generated by the drive signal on a device such as the ultrasonic probe 24 can be suppressed.

According to the first embodiment, as hereinabove described, the ultrasonic probe 24 is provided with the ultrasonic vibrating element 24a detecting the acoustic wave 80 as the ultrasonic wave. Furthermore, in a plan view, the ultrasonic vibrating element 24a is arranged in the substantially central portion of the detecting portion 20, and the light source portions 21 are arranged adjacent to the ultrasonic vibrating element 24a. Thus, the light source portions 21 can be easily arranged at positions in the vicinity of the ultrasonic probe 24 having the ultrasonic vibrating element 24a.

According to the first embodiment, as hereinabove described, the ultrasonic vibrating element 24a of the ultrasonic probe 24 is arranged to extend along the direction Y, and the light source portions 21 having the LED elements 21a and the LED elements 21b are arranged in the vicinity of the ultrasonic probe 24 to extend along the direction Y. Thus, both the ultrasonic vibrating element 24a detecting the acoustic wave 80 as the ultrasonic wave and the light source portions 21 having the LED elements 21a and the LED elements 21b are arranged along the direction Y, and hence the light can be emitted from the light source portions 21 to the test object 10 along the arrangement of the ultrasonic vibrating element 24a. Consequently, the acoustic wave 80 along the direction Y can be generated, and hence the ultrasonic vibrating element 24a along the direction Y can efficiently detect the generated acoustic wave 80.

According to the first embodiment, as hereinabove described, the light source portions 21 are arranged in the vicinity of the ultrasonic probe 24 to sandwich the ultrasonic vibrating element 24a from the first side (X1 side) of the direction X orthogonal to the direction Y and the second side (X2 side) thereof. Thus, the light along the arrangement of the ultrasonic vibrating element 24a can be emitted to the test object 10 from the first side and the second side of the ultrasonic vibrating element 24a detecting the acoustic wave 80, and hence the amount of light emitted to the test object 10 can be easily increased. Consequently, the larger acoustic wave 80 along the direction Y can be generated, and hence the acoustic wave 80 generated by the ultrasonic vibrating element 24a along the direction Y can be more efficiently detected.

According to the first embodiment, as hereinabove described, the light source portions 21 are arranged such that the mounting surfaces 21c mounted with the LED elements 21a and the LED elements 21b are inclined toward the ultrasonic vibrating element 24a. Thus, the light from the light source portions 21 can be emitted in a direction in which the ultrasonic vibrating element 24a is arranged, and hence the light can be reliably emitted to the test object 10 in the vicinity of the ultrasonic vibrating element 24a. Consequently, the detectable acoustic wave 80 can be more efficiently generated.

According to the first embodiment, as hereinabove described, the light guide portions 26 guiding the light from the light source portions 21 included in the detecting portion 20 to the test object 10 are further provided in the detecting portion 20. Thus, a loss of the light between the light source portions 21 and the test object 10 can be reduced, and hence the loss of the light is reduced, so that the use efficiency of the light emitted from the light source portions 21 can be improved.

According to the first embodiment, as hereinabove described, the light guide portions 26 are formed to extend along the direction Y in which the light source portions 21 extend. Thus, both the light source portions 21 emitting the light and the light guide portions 26 guiding the light from the light source portions 21 to the test object 10 are arranged along the direction Y, and hence the light can be reliably emitted from the light source portions 21 to the light guide portions 26. Consequently, the loss of the light between the light source portions 21 and the test object 10 can be reduced, and hence the loss of the light is reduced, so that the use efficiency of the light emitted from the light source portions 21 can be improved.

According to the first embodiment, as hereinabove described, the drive power supply portion 23 arranged in the vicinity of the light source portions 21 of the detecting portion 20, supplying power to the light source portions 21 is further provided in the detecting portion 20. Thus, the drive power supply portion 23 is arranged in the vicinity of the light source portions 21, and hence the drive power supply portion 23 is close to the light source portions 21, so that generation of noise can be suppressed when the drive power supply portion 23 supplies power to the light source portions 21. Consequently, a bad influence (disturbance of the signal or the like) of the noise generated when the power is supplied on a device such as the ultrasonic probe 24 can be further suppressed.

According to the first embodiment, as hereinabove described, the condensing lenses 25 arranged between the light source portions 21 and the light guide portions 26, condensing the light from the light source portions 21 onto the light guide portions 26 are provided in the detecting portion 20. Thus, the light from the light source portions 21 can be reliably condensed onto the light guide portions 26, and hence the use efficiency of the light is increased, and the amount of light emitted to the test object 10 can be easily ensured.

According to the first embodiment, as hereinabove described, the photoacoustic imaging apparatus 100 is provided with the LED elements 21a arranged in the vicinity of the ultrasonic probe 24, emitting the light having the first wavelength and the LED elements 21b arranged in the vicinity of the ultrasonic probe 24, emitting the light having the second wavelength. Furthermore, the ultrasonic probe 24 is configured to detect the first acoustic wave 80a and the second acoustic wave 80b generated by the absorption of the light having the first wavelength and the light having the second wavelength by the light absorber 10a in the test object 10. Thus, two types of wavelengths are employed, whereby the amount of detected information can be increased (two types of acoustic waves 80a and 80b can be obtained), and hence more various image information (detection signals) can be obtained, as compared with the case where one wavelength is employed.

According to the first embodiment, as hereinabove described, the LED drive circuit 22a driving the LED elements 21a and the LED drive circuit 22b driving the LED elements 21b are provided in the light source drive circuit 22. Furthermore, the LED drive circuit 22a and the LED drive circuit 22b are configured to drive the LED elements 21a and the LED elements 21b such that the output of the light having the first wavelength and the output of the light having the second wavelength are substantially equal to each other. Thus, even when the conversion efficiency of the light output with respect to the power of the LED elements 21a and the conversion efficiency of the light output with respect to the power of the LED elements 21b are different from each other, the LED drive circuit 22a and the LED drive circuit 22b drive the LED elements 21a and the LED elements 21b separately, whereby the outputs of the light having two types of wavelengths can be substantially equal to each other, and hence the intensity of the light having two types of wavelengths emitted to the test object 10 can be matched.

According to the first embodiment, as hereinabove described, the LED elements 21a and the LED elements 21b are arranged in the light source portions 21 such that the distribution shapes of the light emitted from the plurality of the LED elements 21a and the plurality of LED elements 21b are substantially identical to each other. Thus, variations in emission of the light having the first and second wavelengths to the light absorber 10a of the test object 10 resulting from the distribution shapes of the light from the LED elements 21a and the LED elements 21b can be suppressed.

According to the first embodiment, as hereinabove described, the distribution shapes of the light having the first wavelength and the second wavelength emitted from the plurality of LED elements 21a and the plurality of LED elements 21b are substantially identical to each other by alternately arranging the LED elements 21a formed of a plurality of groups and the LED elements 21b formed of a plurality of groups. Thus, not only the distribution shapes of the light from the LED elements 21a and the LED elements 21b but also the distribution positions of the light having the first wavelength and the second wavelength with respect to the light source portions 21 can be substantially identical to each other, and hence the variations in the emission of the light having the first and second wavelengths to the light absorber 10a of the test object 10 resulting from the distribution shapes and the distribution positions of the light from the LED elements 21a and the LED elements 21b can be reliably suppressed.

According to the first embodiment, as hereinabove described, the light source portions 21 are arranged along the direction Y, and the LED elements 21a formed of the plurality of groups and the LED elements 21b formed of the plurality of groups are alternately arranged along the direction Y. Thus, the distribution shapes of the light having the first wavelength and the second wavelength emitted from the plurality of LED elements 21a and the plurality of LED elements 21b can be substantially identical to each other along the direction Y.

According to the first embodiment, as hereinabove described, the ultrasonic probe 24 is configured to generate the ultrasonic wave to the test object 10 and detect the ultrasonic wave reflected by the test object 10. Furthermore, the signal processing portion 30 is configured to process and image the signal on the basis of the detection results of both the acoustic wave 80 generated by the absorption of the light by the light absorber 10a in the test object 10 and the ultrasonic wave 90 reflected by the test object 10. Thus, the signal processing portion 30 can image the detection signals of not only the acoustic wave 80 generated by the absorption of the light by the light absorber 10a in the test object 10 but also the ultrasonic wave 90 reflected by the test object 10, and hence the image in the test object 10 can be more accurately obtained.

According to the first embodiment, as hereinabove described, the signal processing portion 30 is configured to correct the detection result of the ultrasonic probe 24 by the temperature correcting portion 51 and image the detection result on the basis of the temperature information acquired by the temperature sensors 27. Thus, even when the wavelengths of the light emitted from the plurality of LED elements 21a and the plurality of LED elements 21b are varied according to the temperature, the temperature correcting portion 51 can correct the detection result on the basis of the temperature information acquired by the temperature sensors 27, and hence the acoustic wave image can be more accurately obtained.

According to the first embodiment, as hereinabove described, the light sensors 28 are directly connected to the light source drive circuit 22 in the detecting portion 20, and the light source drive circuit 22 is configured to stop the light emission from the light source portions 21 to the test object 10 when the amount of reflection of the light is smaller than the prescribed amount on the basis of the light detection results of the light sensors 28. Thus, the light source drive circuit 22 provided in the detecting portion 20 can promptly turn off (stop) the light emission from the light source portions 21 to the test object 10 when the detecting portion 20 arranged close to the test object 10 is separated from the test object 10 and the amount of reflection of the light is reduced, for example.

### (Second Embodiment)

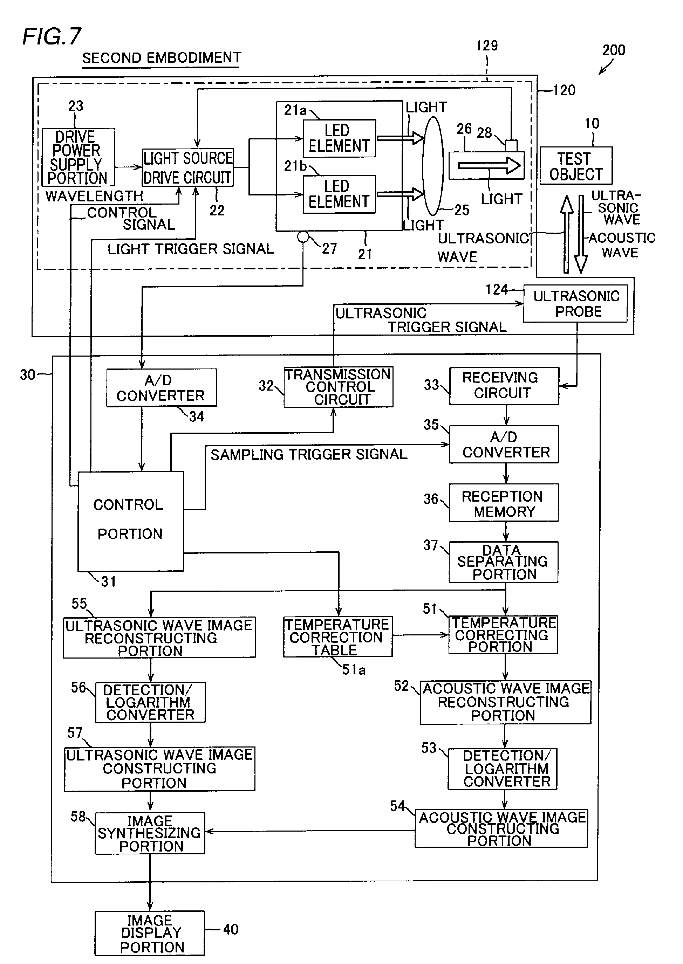

A second embodiment is now described with reference to Figs. 7 to 10. In this second embodiment, a detecting portion 120 is constituted by an ultrasonic probe 124 and a light source unit 129, unlike the aforementioned first embodiment in which the detecting portion 20 is formed by arranging each component such as the light source portions 21 in the ultrasonic probe 24. The ultrasonic probe 124 is an example of the "ultrasonic detector" in the present invention.

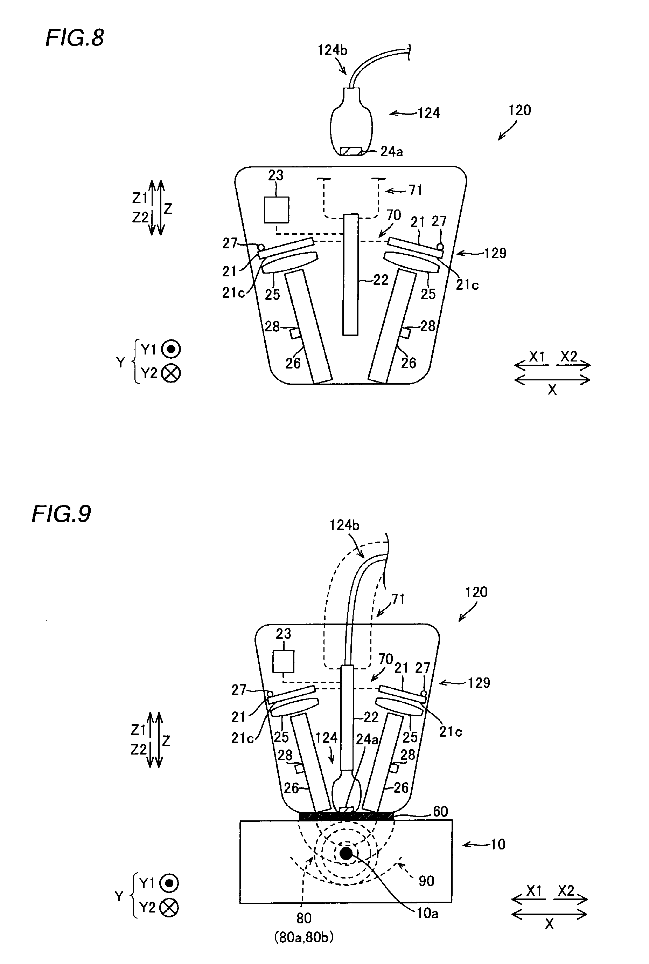

The detecting portion 120 of a photoacoustic imaging apparatus 200 according to the second embodiment of the present invention includes the ultrasonic probe 124 and the light source unit 129 capable of being coupled to the ultrasonic probe 124, as shown in Fig. 7. The light source unit 129 includes a light source portion 21 having a plurality of LED elements 21a and a plurality of LED elements 21b, a light source drive circuit 22 transmitting a drive signal to the light source portion 21, a drive power supply portion 23 supplying power, a condensing lens 25 condensing light, a light guide portion 26 guiding light to the vicinity of a test object 10, a temperature sensor 27 detecting the temperature of the light source portion 21, and a light sensor 28 detecting light reflected by the test object 10. In Fig. 7, the detecting portion 120 includes only a set of the light source portion 21, the condensing lens 25, the light guide portion 26, the temperature sensor 27, and the light sensor 28 for the convenience of illustration, but two sets of the aforementioned components are actually included in the detecting portion 120, as shown in Fig. 8.

According to the second embodiment, the light source drive circuit 22 is configured to supply power to both the LED elements 21a and the LED elements 21b and drive the same. The light source drive circuit 22 is configured to receive a wavelength control signal and a light trigger signal from a control portion 31 of a signal processing portion 30 and drive the LED elements 21a and the LED elements 21b on the basis of the received signals.

According to the second embodiment, the detecting portion 120 is constituted by the ultrasonic probe 124 (an ultrasonic vibrating element 24a detachably provided) separately provided and the light source unit 129, as shown in Fig. 8. Fig. 8 shows a state before the ultrasonic probe 124 and the light source unit 129 are coupled to each other. The ultrasonic probe 124 and the light source unit 129 can be coupled to each other in any manner. For example, the ultrasonic probe 124 and the light source unit 129 may have coupling portions and be coupled to each other by coupling the coupling portions to each other, or the ultrasonic probe 124 and the light source unit 129 may be coupled (fixed) to each other by a member such as a screw.

The ultrasonic probe 124 has the ultrasonic vibrating element 24a configured to detect an acoustic wave 80 and an ultrasonic wave 90 in the vicinity of an end face on a Z2 side. The ultrasonic probe 124 is connected to the signal processing portion 30 by a wire 124b. Furthermore, the ultrasonic probe 124 is configured to receive an ultrasonic trigger signal from the signal processing portion 30 through the wire 124b and transmit a detected acoustic wave signal and a detected ultrasonic signal to the signal processing portion 30.

As shown in Fig. 9, in a state where the ultrasonic probe 124 and the light source unit 129 are coupled to each other, the ultrasonic probe 124 is arranged in the vicinity of the center of the light source unit 129 in a direction X. The ultrasonic vibrating element 24a of the ultrasonic probe 124 is arranged in the vicinity of the end face closer to the test object 10 (on the Z2 side) in the detecting portion 120. In the detecting portion 120, the ultrasonic probe 124 is arranged to be sandwiched between the two light source portions 21, the two condensing lenses 25, and the two light guide portions 26. The detecting portion 120 is configured to emit light from the light source portions 21 to the test object 10 in the state where the ultrasonic probe 124 and the light source unit 129 are coupled to each other.

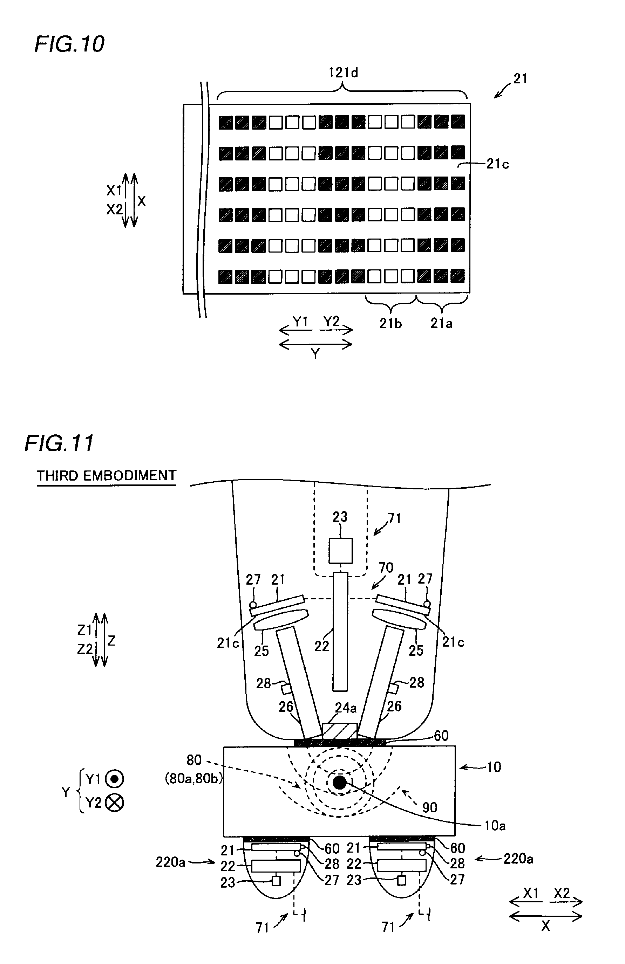

In the light source portions 21, the LED elements 21a having a first wavelength and the LED elements 21b having a second wavelength are arranged in a prescribed arrangement pattern 121d on a mounting surface 21c, as shown in Fig. 10. In the prescribed arrangement pattern 121d, the number of the arranged LED elements 21a and the number of the arranged LED elements 21b are determined according to the maximum output ratio of the light emitted from the LED elements 21a and the LED elements 21b such that the total output of the light from the plurality of LED elements 21a and the total output of the light from the plurality of LED elements 21b are substantially equal to each other. As an example, the case where the LED elements 21a have a maximum output 2W and the LED elements 21b have a maximum output 3W is described. In this case, the ratio of the number of the arranged LED elements 21a and the number of the arranged LED elements 21b is equal to the ratio of the maximum output of the LED elements 21a and the maximum output of the LED elements 21b. In other words, the number of the arranged LED elements 21a and the number of the arranged LED elements 21b are adjusted such that the ratio of the number of the arranged LED elements 21a to the number of the arranged LED elements 21b is 3:2 in the arrangement pattern 121d. For the convenience of illustration, only one arrangement pattern 121d is shown in Fig. 10, but a prescribed number of the arrangement patterns 121d is actually arranged on the mounting surface 21c sequentially.

The remaining structure of the photoacoustic imaging apparatus 200 according to the second embodiment is similar to that of the photoacoustic imaging apparatus 100 according to the aforementioned first embodiment.

According to the second embodiment, the following effects can be obtained.

According to the second embodiment, as hereinabove described, the light source portions 21 each are provided with the plurality of LED elements 21a and the plurality of LED elements 21b, the numbers of which are adjusted according to the ratio of the maximum output of the light emitted from the LED elements 21a and the maximum output of the light emitted from the LED elements 21b such that the total output of the light from the plurality of LED elements 21a and the total output of the light from the plurality of LED elements 21b are substantially equal to each other. Thus, even when the conversion efficiency of the light output with respect to the power of the LED elements 21a and the conversion efficiency of the light output with respect to the power of the LED elements 21b are different from each other, the number of the plurality of LED elements 21a and the number of the plurality of LED elements 21b are adjusted according to the ratio of the maximum outputs, whereby the outputs of the light having two types of wavelengths can be substantially equal to each other, and hence the intensity of the light having two types of wavelengths emitted to the test object 10 can be matched.

According to the second embodiment, as hereinabove described, the ultrasonic vibrating element 24a is detachably provided in the detecting portion 120. Thus, even when the ultrasonic vibrating element 24a is broken, the entire detecting portion 120 may not be replaced, and hence the detecting portion 120 can be easily repaired.

The remaining effects of the second embodiment are similar to those of the aforementioned first embodiment.

### (Third Embodiment)

A third embodiment is now described with reference to Fig. 11. In this third embodiment, auxiliary emitting portions 220a each including a light source portion 21 are provided separately from a detecting portion 20, unlike each of the aforementioned first and second embodiments in which the light source portions 21 are provided in the detecting portion 20 (120).

A photoacoustic imaging apparatus 300 (see Fig. 1) according to the third embodiment of the present invention includes the detecting portion 20 and the two auxiliary light emitting portions 220a provided separately from the detecting portion 20, as shown in Fig. 11.

According to the third embodiment, the auxiliary light emitting portions 220a each include the light source portion 21 having LED elements 21a and LED elements 21b, a light source drive circuit 22 transmitting a drive signal to the light source portion 21, a drive power supply portion 23 supplying power, a temperature sensor 27 detecting the temperature of the light source portion 21, and a light sensor 28 detecting light reflected by a test object 10.

According to the third embodiment, the auxiliary light emitting portions 220a are opposed to the detecting portion 20 to sandwich the test object 10 between the auxiliary light emitting portions 220a and the detecting portion 20. The auxiliary light emitting portions 220a are configured to emit light to the test object 10 in a state where transparent gel layers 60 transmitting light intervene between the auxiliary light emitting portions 220a and the test objet 10. Furthermore, the auxiliary light emitting portions 220a are configured to emit the light from the light source portions 21 to the test object 10 through the gel layers 60. The auxiliary light emitting portions 220a are configured to emit the light in synchronization with the timing of light emission from the detecting portion 20.

The remaining structure of the photoacoustic imaging apparatus 300 according to the third embodiment is similar to that of the photoacoustic imaging apparatus 100 according to the aforementioned first embodiment.

According to the third embodiment, the following effects can be obtained.

According to the third embodiment, as hereinabove described, the photoacoustic imaging apparatus 300 is provided with light source portions 21 having a plurality of LED elements 21a and a plurality of LED elements 21b emitting the light to the test object 10, whereby no optical surface plate for suppressing property fluctuation resulting from vibration of an optical system (device peripheral to a solid-state laser beam source) or strong housing is necessary unlike the case where a solid-state laser is employed as a light source, and hence the light source portions 21 can be downsized. Furthermore, the light source portions 21 are provided in the detecting portion 20 arranged close to the test object 10, whereby the light source portions 21 and the detecting portion 20 are integrated, so that the simple structure can be achieved. Thus, the photoacoustic imaging apparatus 300 can be downsized and simplified. In addition, the photoacoustic imaging apparatus 300 is provided with the auxiliary light emitting portions 220a, whereby not only the light from the detecting portion 20 but also the light from the auxiliary light emitting portions 220a can be emitted to the test object 10, and hence an acoustic wave 80 can be obtained at a prescribed intensity even when the light is easily attenuated in the test object 10 or even when the thickness of the test object 10 is large. Consequently, this photoacoustic imaging apparatus 300 can be applied to more various test objects.

The remaining effects of the third embodiment are similar to those of the aforementioned first embodiment.

The embodiments disclosed this time must be considered as illustrative in all points and not restrictive. The range of the present invention is shown not by the above description of the embodiments but by the scope of claims for patent, and all modifications within the meaning and range equivalent to the scope of claims for patent are further included.

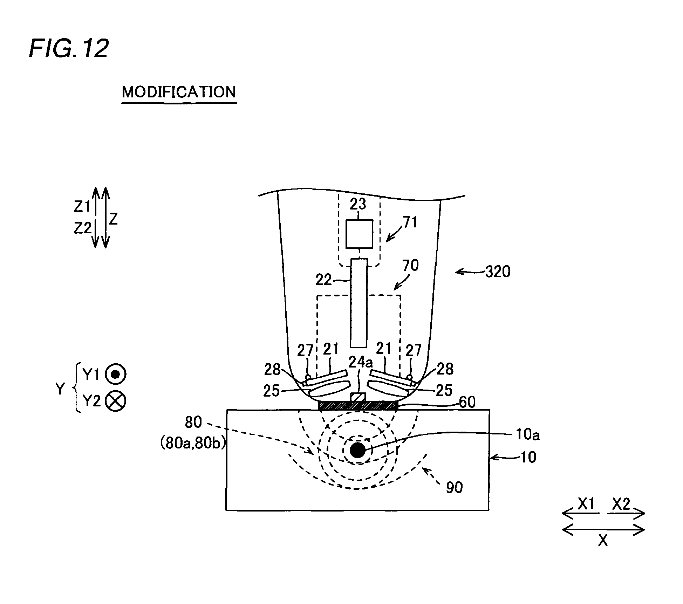

For example, while the light is emitted from the light source portions 21 to the test object 10 through the light guide portions 26 in each of the aforementioned first to third embodiments, the present invention is not restricted to this. According to the present invention, the light may alternatively be emitted from the light source portions 21 to the test object 10 without providing the light guide portions 26. As in a modification shown in Fig. 12, for example, light source portions 21 and condensing lenses 25 of a detecting portion 320 may be arranged in the vicinity of a test object 10, and light condensed by the condensing lenses 25 may be emitted to the test object 10.

While the light source drive circuit 22 and the drive power supply portion 23 are arranged in the detecting portion 20 (120) in order to suppress the generation of noise in each of the aforementioned first to third embodiments, the light source drive circuit 22 and the drive power supply portion 23 are preferably shielded and arranged in the detecting portion 20 (120) according to the present invention. Furthermore, the wires of the light source drive circuit 22 and the drive power supply portion 23 are more preferably shielded.

While the drive power supply portion 23 is arranged in the detecting portion 20 (120) in each of the aforementioned first to third embodiments, the present invention is not restricted to this. According to the present invention, the drive power supply portion 23 may alternatively be arranged in a place other than the detecting portion 20 (120).

While one condensing lens is provided with respect to one light source portion 21 in each of the aforementioned first to third embodiments, the present invention is not restricted to this. According to the present invention, a plurality of condensing lenses may alternatively be provided with respect to one light source portion 21. According to this structure, the thickness of one lens can be reduced.

While the light sensors 28 are arranged on the light guide portions 26 in each of the aforementioned first to third embodiments, the present invention is not restricted to this. According to the present invention, the light sensors 28 may alternatively be arranged in any place, so far as the same can detect the light reflected by the test object 10. For example, the light sensors 28 may be provided on a housing forming the outer shape of the detecting portion 20 (120).

While the light source drive circuit 22 has the two LED drive circuits 22a and 22b with different drive power in the aforementioned first embodiment, the present invention is not restricted to this. According to the present invention, one LED drive circuit may alternatively supply drive power to the LED elements 21a and the LED elements 21b, and the drive power supplied to the LED elements 21a and the LED elements 21b may alternatively be made different by the wavelength control signal.

While the ultrasonic probe 24 (124) detects the acoustic wave 80 and the ultrasonic wave 90 reflected inside the test object 10 and generates the ultrasonic wave 90 to the test object 10 in each of the aforementioned first to third embodiments, the present invention is not restricted to this. According to the present invention, a device other than the ultrasonic probe may alternatively generate the ultrasonic wave.

While the ultrasonic probe 24 (124) generates the ultrasonic wave 90 in each of the aforementioned first to third embodiments, the present invention is not restricted to this. According to the present invention, the ultrasonic probe 24 (124) may alternatively detect the acoustic wave 80 without generating the ultrasonic wave 90.

## Claims

1. A photoacoustic imaging apparatus (100, 200, 300) comprising:
a detecting portion (20, 120, 320) configured to be capable of emitting light to a test object (10), detecting an acoustic wave (80) generated by absorption of the light that is emitted by a target (10a) in the test object; and
a signal processing portion (30) processing and imaging a signal detected by the detecting portion,
the detecting portion arranged close to the test object, including a light source portion (21) having a plurality of LED elements (21a, 21b) emitting the light to the test object, an ultrasonic detector (24, 124) arranged in a vicinity of the light source portion, configured to detect the acoustic wave as an ultrasonic wave generated by the absorption of the light emitted from the light source portion to the test object, and a light source drive circuit (22) arranged in the vicinity of the light source portion, driving the light source portion.

2. The photoacoustic imaging apparatus according to claim 1, wherein
the ultrasonic detector has an ultrasonic vibrating element (24a) detecting the acoustic wave as the ultrasonic wave, and
in a plan view, the ultrasonic vibrating element is arranged in a central portion of the detecting portion, and the light source portion is arranged adjacent to the ultrasonic vibrating element.

3. The photoacoustic imaging apparatus according to claim 2, wherein
the ultrasonic vibrating element of the ultrasonic detector is arranged to extend along a first direction, and
the light source portion having the LED elements is arranged in a vicinity of the ultrasonic detector to extend along the first direction.

4. The photoacoustic imaging apparatus according to claim 3, wherein
the light source portion is arranged in the vicinity of the ultrasonic detector to sandwich the ultrasonic vibrating element from a first side of a second direction orthogonal to the first direction and a second side thereof.

5. The photoacoustic imaging apparatus according to any of claims 2 to 4, wherein
the light source portion is arranged such that a mounting surface mounted with the LED elements is inclined toward the ultrasonic vibrating element.

6. The photoacoustic imaging apparatus according to any of claims 1 to 5, wherein
the detecting portion further includes a light guide portion (26) guiding the light from the light source portion included in the detecting portion to the test object.

7. The photoacoustic imaging apparatus according to claim 6, wherein
the light source portion is arranged to extend along a first direction, and
the light guide portion is formed to extend along the first direction in which the light source portion extends.

8. The photoacoustic imaging apparatus according to any of claims 1 to 7, wherein
the detecting portion further includes a drive power supply portion (23) arranged in the vicinity of the light source portion of the detecting portion, supplying power to the light source portion.

9. The photoacoustic imaging apparatus according to claim 6 or 7, wherein
the detecting portion further includes a condensing portion (25) arranged between the light source portion and the light guide portion, condensing the light from the light source portion onto the light guide portion.

10. The photoacoustic imaging apparatus according to any of claims 1 to 9, wherein
the plurality of LED elements of the light source portion include a first LED element (21a) arranged in a vicinity of the ultrasonic detector, emitting the light having a first wavelength and a second LED element (21b) arranged in the vicinity of the ultrasonic detector, emitting the light having a second wavelength.

11. The photoacoustic imaging apparatus according to claim 10, wherein
the light source drive circuit is configured to drive the first LED element and the second LED element such that an output of the light having the first wavelength and an output of the light having the second wavelength are equal to each other.

12. The photoacoustic imaging apparatus according to claim 10 or 11, wherein
the light source portion has a plurality of first LED elements and a plurality of second LED elements, numbers of which are adjusted according to a ratio of a maximum output of the light emitted from the first LED elements and a maximum output of the light emitted from the second LED elements such that a total output of the light from the plurality of first LED elements and a total output of the light from the plurality of second LED elements are equal to each other.

13. The photoacoustic imaging apparatus according to any of claims 1 to 12, wherein
the first LED element and the second LED element are arranged in the light source portion such that distribution shapes of the light emitted from a plurality of first LED elements and a plurality of second LED elements are identical to each other.

14. The photoacoustic imaging apparatus according to claim 13, wherein
the light source portion is configured such that the distribution shapes of the light having the first wavelength and the second wavelength emitted from the plurality of LED elements are identical to each other by alternately arranging the plurality of first LED elements formed of a single group or a plurality of groups and the plurality of second LED elements formed of a single group or a plurality of groups.

15. The photoacoustic imaging apparatus according to any of claims 1 to 14, wherein
the ultrasonic detector is configured to generate the ultrasonic wave to the test object and detect the ultrasonic wave (90) reflected by the test object, and
the signal processing portion is configured to process and image the signal on the basis of detection results of both the acoustic wave generated by the absorption of the light by the target in the test object and the ultrasonic wave reflected by the test object.
